# EUROPÄISCHE PATENTSCHRIFT

(11) **EP 2 731 653 B1**
(45) Veröffentlichungstag und Bekanntmachung des Hinweises auf die Patenterteilung: **07.10.2015**
(21) Anmeldenummer: 12737718.2
(22) Anmeldetag: 13.07.2012
(51) Int. Cl.: A61M 5/46

(54) **INJEKTIONSGERÄT UND VERFAHREN ZU DESSEN HERSTELLUNG**
INJECTION DEVICE AND METHOD FOR PRODUCING SAME
DISPOSITIF D'INJECTION ET PROCÉDÉ DE FABRICATION DE CE DISPOSITIF

(30) Priorität: 13.07.2011 DE 102011107199
(43) Veröffentlichungstag der Anmeldung: 21.05.2014
(73) Patentinhaber: Haselmeier AG, 9001 St. Gallen (CH)
(72) Erfinder: KEITEL, Joachim, 73728 Esslingen (DE)
(74) Vertreter: Reinhardt, Annette
(86) Internationale Anmeldenummer: PCT/EP2012/002964
(87) Internationale Veröffentlichungsnummer: WO 2013/007393

(56) Entgegenhaltungen:
- EP-B1- 0 762 904
- WO-A1-99/27986
- WO-A1-2009/155277
- WO-A2-2006/063015
- WO-A2-2008/048750

## Beschreibung

Die Erfindung betrifft ein Injektionsgerät und ein Verfahren zu dessen Herstellung.

Aus der EP 0 762 904 B1 ist ein Injektionsgerät bekannt, bei dem ein Halter, der eine Injektionsnadel trägt und in dem eine Karpule mit Injektionsflüssigkeit angeordnet ist, relativ zum Gehäuse bewegt wird. Durch das Zurückziehen des Halters ist die Injektionsnadel vor dem Injektionsvorgang im Gehäuse verborgen. Dies verringert die Angst des Benutzers vor der Nadel.

Zur Herstellung des Injektionsgeräts ist es aus der EP 0 762 904 B1 bekannt, den Halter für die Karpule mit Injektionsflüssigkeit so an der Antriebseinrichtung anzuordnen, dass der Stößel, der zum Ausdrücken von Injektionsflüssigkeit aus dem Behälter dient, vor der ersten Injektion an dem Stopfen der Karpule anliegt. Zur Fixierung des Halters ist bei der EP 0 762 904 B1 eine Mikrorastung vorgesehen.

Die Lage des Stopfens in der Karpule unterliegt produktionsbedingt vergleichsweise hohen Toleranzen. Je nach Lage des Stopfens ergibt sich damit eine andere Lage der Injektionsnadel in dem Gehäuse. Da der Weg, den der Behälter gegenüber dem Gehäuse zurücklegt, konstruktiv vorgegeben ist, verändert sich in Abhängigkeit der Lage der Injektionsnadel die Einstichtiefe. Zur Regulierung der Einstichtiefe ist aus der EP 0 762 904 B1 eine Hülse bekannt, mit der die Einstichtiefe vom Bediener manuell verändert werden kann.

Für bestimmte Anwendungen ist es vorteilhaft, die Injektion mit einer genau definierten Einstichtiefe durchzuführen. Dies ist insbesondere dann zweckmäßig, wenn die Injektion mit einer sehr kurzen Injektionsnadel durchgeführt wird. Bei sehr kurzen Injektionsnadeln muss zum einen sichergestellt werden, dass eine ausreichende Einstichtiefe erreicht wird, damit die Injektionsflüssigkeit sicher injiziert wird. Zum anderen muss gewährleistet sein, dass der Nadelhalter nicht zu weit aus dem Gehäuse austritt.

Der Erfindung liegt die Aufgabe zugrunde, ein Verfahren zur Herstellung eines Injektionsgeräts bereitzustellen, mit dem eine gewünschte Einstichtiefe sichergestellt werden kann. Eine weitere Aufgabe der Erfindung besteht darin, ein Injektionsgerät bereitzustellen, das eine definierte Einstichtiefe besitzt.

Diese Aufgabe wird bezüglich des Verfahrens durch ein Verfahren mit den Merkmalen des Anspruchs 1 gelöst. Bezüglich des Injektionsgeräts wird die Aufgabe durch ein Injektionsgerät mit den Merkmalen des Anspruchs 7 gelöst.

Das Injektionsgerät besitzt ein mehrteiliges Gehäuse, das mindestens ein erstes und ein zweites Gehäuseteil umfasst. Diese beiden Gehäuseteile werden bei der Herstellung des Injektionsgeräts relativ zueinander positioniert. Die Positionierung erfolgt dabei so, dass die Aufnahme in ihrer proximalen Endlage eine vorgegebene Lage relativ zu der Auflage besitzt. Das erste Gehäuseteil wird dann in dieser Position gegenüber dem zweiten Gehäuseteil unlösbar fixiert. Das erste Gehäuseteil wird insbesondere direkt an dem zweiten Gehäuseteil fixiert. Das erste Gehäuseteil kann jedoch auch an einem fest mit dem zweiten Gehäuseteil verbundenen Bauteil fixiert werden. Unter einer unlösbaren Fixierung wird eine Fixierung verstanden, die nicht vom Benutzer gelöst werden kann, ohne das Injektionsgerät zu beschädigen. Dadurch kann die einmal bei der Herstellung durch Positionierung der beiden Gehäuseteile zueinander eingestellte Einstichtiefe vom Bediener nicht verändert werden. Aufgrund der Positionierung der beiden Gehäuseteile zueinander können Fertigungstoleranzen zwischen den Einzelteilen des Injektionsgeräts ausgeglichen werden, und die Einstichtiefe kann im Rahmen der Genauigkeit der Positionierung der beiden Gehäuseteile zueinander festgelegt werden.

Die vorliegende Erfindung ist für Injektionsgeräte vorgesehen, die nicht nachfüllbar sind. Derartige Injektionsgeräte besitzen einen Behälter mit Injektionsflüssigkeit, nämlich eine sogenannte Karpule oder Kartusche. Die Injektionsflüssigkeit ist für mehrere Injektionen ausreichend. Nach der letzten Injektion wird das Injektionsgerät entsorgt.

Zum Auspressen der Injektionsflüssigkeit ist in dem Behälter ein beweglicher Stopfen angeordnet. Die Antriebseinrichtung besitzt vorteilhaft einen Stößel, der auf den Stopfen wirkt. Über den Stößel kann Injektionsflüssigkeit aus dem Behälter herausgedrückt werden. Damit schon bei der ersten Injektion eine definierte Menge an Injektionsflüssigkeit injiziert wird, ist vorgesehen, dass der Behälter relativ zu dem Stößel so positioniert wird, dass sich eine definierte Lage des Stopfens relativ zu dem Stößel ergibt, und dass der Behälter in dieser Position fixiert wird. Dadurch kann vermieden werden, dass der Benutzer zunächst eine geringe Menge Injektionsflüssigkeit verwerfen muss, um sicherzustellen, dass der Stößel am Stopfen anliegt. Der Behälter kann so positioniert werden, dass der Stößel am Stopfen anliegt, es kann jedoch auch vorteilhaft sein, wenn ein definierter Abstand zwischen dem Stößel und dem Stopfen eingestellt wird. Dieser definierte Abstand muss konstruktiv beim Dosierweg für die erste Injektion berücksichtigt werden.

Vorteilhaft erfolgt die Positionierung des Behälters relativ zu dem Stößel vor der Positionierung des ersten Gehäuseteils relativ zu dem zweiten Gehäuseteil. Über die Positionierung des ersten Gehäuseteils am zweiten Gehäuseteil können damit die Toleranzen der Lage des Stopfens ausgeglichen werden. Dadurch kann zum einen eine sehr genaue Einstichtiefe erreicht werden und zum anderen ist die Verwendung von Behältern mit Injektionsflüssigkeit möglich, bei denen die Lage des Stopfens weniger genau definiert ist. Dadurch wird die Herstellung vereinfacht.

Das Injektionsgerät besitzt vorteilhaft ein Koppelelement, das mit dem Stößel zusammenwirkt. Der Behälter wird nach der Positionierung relativ zu dem Stößel vorteilhaft gegenüber dem Koppelelement fixiert. Dadurch kann auf einfache Weise eine Positionierung des Behälters gegenüber dem Stößel erfolgen. Insbesondere wird der Behälter in einem Halter angeordnet, der die Aufnahme trägt, und der Halter wird mit dem darin angeordneten Behälter an dem Koppelelement fixiert. Über den Halter und das Koppelelement wird damit die Aufnahme relativ zu der Antriebseinrichtung positioniert.

Eine einfache Herstellung ergibt sich, wenn das erste Gehäuseteil mit dem zweiten Gehäuseteil verklebt wird. Vorteilhaft wird ein Klebstoff verwendet, der durch Strahlung, insbesondere durch UV-Strahlung, aushärtet. Es kann jedoch auch vorgesehen sein, dass das erste Gehäuseteil mit dem zweiten Gehäuseteil verpresst wird. Besonders vorteilhaft erfolgt die Verpressung durch Heißpressen. Es kann auch vorgesehen sein, dass das erste mit dem zweiten Gehäuseteil verschweißt wird. Vorteilhaft erfolgt das Verschweißen durch Laserschweißen oder Ultraschallschweißen.

Ein Injektionsgerät, das eine definierte Einstichtiefe sicherstellt, besitzt ein Gehäuse mit einem ersten und einem zweiten Gehäuseteil, wobei das erste Gehäuseteil unlösbar gegenüber dem zweiten Gehäuseteil fixiert ist. Das erste Gehäuseteil ist insbesondere direkt an dem zweiten Gehäuseteil fixiert. Die mehrteilige Ausbildung des Gehäuses erlaubt eine genaue Positionierung der Gehäuseteile relativ zueinander, so dass die Einstichtiefe genau eingestellt werden kann. Dadurch, dass die beiden Gehäuseteile fest miteinander verbunden sind, ist eine spätere Veränderung der Einstichtiefe durch den Bediener ausgeschlossen.

Um sicherzustellen, dass bereits bei der ersten Injektion eine voreingestellte Menge Injektionsflüssigkeit injiziert wird, ist vorgesehen, dass der Stößel an dem beweglichen Stopfen des Behälters anliegt. Es kann jedoch auch vorgesehen sein, dass der Stößel zu dem Stopfen vor der ersten Injektion einen Abstand besitzt, und dass die Antriebseinrichtung so ausgebildet ist, dass der Stößel bei der ersten Injektion einen Weg zurücklegt, der um den Abstand zwischen Stößel und Stopfen größer als der zum Herausdrücken der eingestellten Menge an Injektionsflüssigkeit benötigte Weg ist. Dies kann durch entsprechende Vergrößerung des Stellwegs für die erste Injektion erfolgen.

Vorteilhaft ist der Behälter mit einem Koppelelement verbunden, das mit dem Stößel zusammenwirkt. Das Koppelelement fixiert den Stößel je nach Betriebszustand relativ zu dem Behälter oder gibt den Stößel gegenüber dem Behälter frei. Unterschiedliche Betriebszustände sind dabei die erste Phase des Spannens des Injektionsgeräts, in der die Injektionsnadel ins Gehäuse zurückgezogen wird, die zweite Phase des Spannens des Injektionsgeräts, in der die Antriebseinrichtung gegenüber dem Stößel um den Weg verstellt wird, der der zu injizierenden Flüssigkeitsmenge entspricht, das Einstechen der Nadel sowie das Injizieren der Injektionsflüssigkeit.

Der Behälter ist vorteilhaft in einem Halter gehalten, der die Aufnahme für die Injektionsnadel besitzt. Dadurch kann der Behälter selbst einfach aufgebaut sein. Vorteilhaft besteht der Halter aus einem durchsichtigen Material. Dadurch kann der Benutzer bei entsprechender Gehäusegestaltung durch den Halter erkennen, wie viel Injektionsflüssigkeit noch im Behälter vorhanden ist. Aufgrund der durchsichtigen Ausbildung des Halters kann der Halter mit dem Koppelelement über eine durch Strahlung, insbesondere durch UV-Strahlung aushärtende Klebverbindung verbunden sein. Dadurch kann auf einfache Weise eine feste, unlösbare Verbindung von Halter und Koppelelement erreicht werden. Der Halter und das Koppelelement bestehen insbesondere aus Kunststoff.

Es ist vorgesehen, dass das Injektionsgerät eine Spannfeder besitzt, die beim Injektionsvorgang sowohl eine Bewegung des Behälters relativ zum Gehäuse als auch eine Bewegung des Stößels relativ zu dem Behälter bewirkt. Dadurch kann mit einer Spannfeder sowohl das Einstechen der Injektionsnadel als auch das Injizieren der Injektionsflüssigkeit bewirkt werden. Dadurch ergibt sich ein einfacher Aufbau des Injektionsgeräts. Vorteilhaft ist die Antriebseinrichtung über eine Koppeleinrichtung mit dem Behälter und dem Stößel verbunden. Die Koppeleinrichtung ist dabei insbesondere in Abhängigkeit der Position von Antriebseinrichtung, Behälter und Stößel relativ zu dem Gehäuse gesteuert. Demnach ist eine wegabhängige Steuerung der Koppeleinrichtung vorgesehen. Die Koppeleinrichtung kann insbesondere als nockengesteuerte Klinkenkupplung ausgeführt sein. Die Koppeleinrichtung steuert vorteilhaft eine erste Verbindung zwischen dem Behälter und dem Stößel und eine zweite Verbindung zwischen dem Stößel und der Antriebseinrichtung. Durch entsprechende Ansteuerung der beiden Verbindungen kann die Koppeleinrichtung auch den Behälter mit der Antriebseinrichtung verbinden.

Ausführungsbeispiele der Ereindung werden im Folgenden anhand der Zeichnung erläutert. Es zeigen:
- Fig. 1: eine Seitenansicht eines Injektionsgeräts,
- Fig. 2: eine schematische Schnittdarstellung durch das Injektionsgerät im Ruhezustand,
- Fig. 3: eine Schnittdarstellung entsprechend Fig. 2 nach dem Zurückziehen der Nadel,
- Fig. 4: eine Schnittdarstellung entsprechend Fig. 2 nach dem vollständigen Spannen der Spannfeder,

- Fig. 5: eine Schnittdarstellung entsprechend Fig. 2 nach dem Einstechen der Injektionsnadel,
- Fig. 6: eine Schnittdarstellung entsprechend Fig. 2 nach dem Injizieren der Injektionsflüssigkeit,
- Fig. 7: einen Schnitt durch das Injektionsgerät in Fig. 1 nach dem Zurückziehen des Behälters und ohne Injektionsnadel,
- Fig. 8: eine ausschnittsweise Darstellung des Bereichs der Aufnahme des Injektionsgeräts aus Fig. 7 mit daran angeordneter Injektionsnadel,
- Fig. 9: einen Schnitt entlang der Linie IX-IX in Fig. 1,
- Fig. 10: das Injektionsgerät aus Fig. 1 vor der Montage des Halters,
- Fig. 11: einen Schnitt entlang der Linie XI-XI in Fig. 10,
- Fig. 12: eine Seitenansicht des Injektionsgeräts aus Fig. 10 nach der Montage des Halters,
- Fig. 13: einen Schnitt entlang der Linie XIII-XIII in Fig. 12,
- Fig. 14: eine Seitenansicht des Injektionsgeräts aus Fig. 10 nach der Montage des ersten Gehäuseteils,
- Fig. 15: einen Schnitt entlang der Linie XV-XV in Fig. 14,
- Fig. 16: einen Längsschnitt durch ein Ausführungsbeispiel eines Injektionsgeräts.

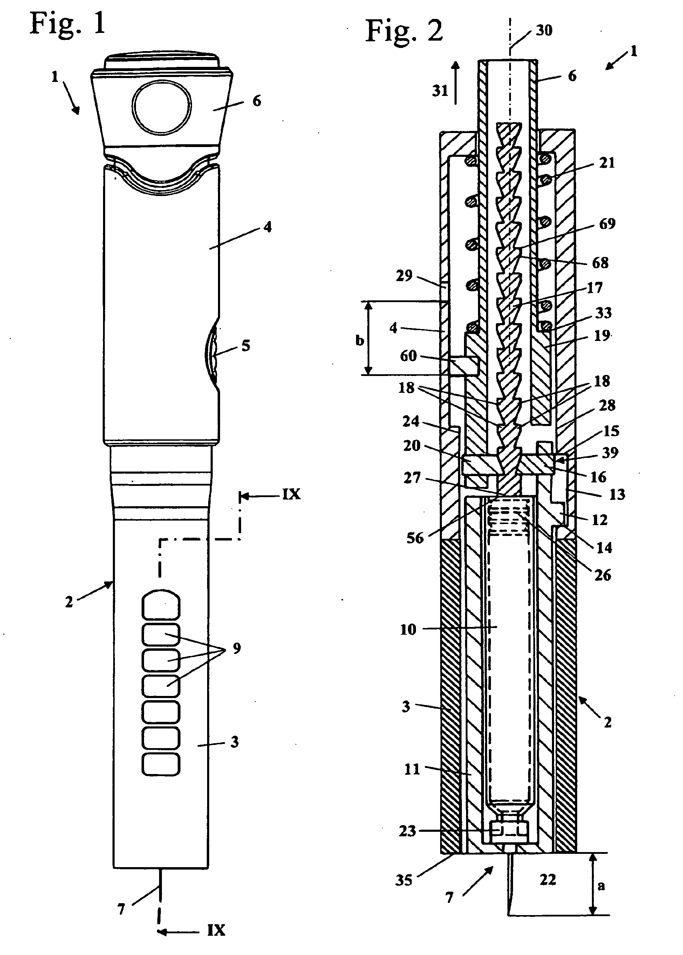

Fig. 1 zeigt ein Injektionsgerät 1. Das Injektionsgerät 1 hat etwa die Form eines großen Füllfederhalters oder Kugelschreibers und dient dazu, eine Injektionsflüssigkeit zu injizieren. Das in Fig. 1 gezeigte Injektionsgerät ist nicht nachfüllbar. Mit dem Injektionsgerät 1 kann eine vorgegebene Anzahl von Injektionen durchgeführt werden. Die pro Injektion zu injizierende Flüssigkeitsmenge ist dabei konstruktiv vorgegeben. Es sind jedoch auch Injektionsgeräte bekannt, beispielsweise aus der EP 0 762 904 B1, bei denen die zu injizierende Flüssigkeitsmenge vom Bediener einstellbar ist. Die vorliegende Erfindung ist auch für Injektionsgeräte vorgesehen, bei denen die zu injizierende Menge einstellbar ist.

Das Injektionsgerät 1 besitzt ein Gehäuse 2, das zweiteilig ausgebildet ist. Das Gehäuse 2 besitzt ein erstes, proximales Gehäuseteil 3 und ein zweites, distales Gehäuseteil 4. Mit "proximal" wird dabei die Seite bezeichnet, die bei einer Injektion der Einstichstelle zugewandt liegt und mit "distal" die Seite, die der Einstichstelle abgewandt liegt. Bei der in Fig. 1 gezeigten, unbetätigten Stellung des Injektionsgeräts 1 ragt eine Injektionsnadel 7 aus dem ersten Gehäuseteil 3. Das erste Gehäuseteil 3 besitzt mehrere Sichtfenster 9, die als Öffnungen in dem aus einem undurchsichtigen Kunststoff hergestellten Gehäuseteil 3 ausgebildet sind. Durch die Sichtfenster 9 ist der Behälter mit Injektionsflüssigkeit sichtbar.

Das Injektionsgerät 1 besitzt zur Bedienung an seiner distalen Seite ein Spannelement 6, das vom Bediener zum Spannen des Injektionsgeräts 1 aus dem Gehäuse 2 herausgezogen werden kann, sowie ein Auslöseelement 5, das als Bedienknopf ausgebildet und am zweiten Gehäuseteil 4 angeordnet ist. Zum Auslösen einer Injektion setzt der Bediener das gespannte Injektionsgerät 1 auf die Haut auf und betätigt das Auslöseelement 5.

Die Funktion des Injektionsgeräts 1 wird anhand der schematischen Darstellungen in den Figuren 2 bis 6 erläutert. Die gezeigte konstruktive Gestaltung dient lediglich der einfachen Erläuterung. Fig. 2 zeigt das Injektionsgerät 1 in unbetätigtem Zustand. Die Injektionsnadel 7 besitzt einen Kanülenhalter 23, an dem eine Kanüle 22 festgelegt ist. Die Kanüle 22 ragt aus dem Gehäuse 2 mit einer Länge a heraus, die der Einstichtiefe entspricht. Beim Injektionsvorgang wird das Gehäuse 2 mit seiner proximalen Stirnseite, die eine Auflage 35 bildet, auf die Haut aufgesetzt.

Im Gehäuse 2 ist im Bereich des ersten Gehäuseteils 3 ein Behälter 10 angeordnet. Der Behälter 10 ist vorteilhaft als Karpule ausgebildet und besitzt an seinem distalen Ende einen Stopfen 26, der zum Auspressen von Injektionsflüssigkeit durch die am proximalen Ende des Behälters 10 angeordnete Kanüle 22 in proximaler Richtung bewegt wird. Der Stopfen 26 besitzt an seinem distalen Ende eine Stirnseite 56, an der ein Stößel 17 mit einer Stirnseite 27 anliegt. Der Stößel 17 dient zur Bewegung des Stopfens 26. Der Stößel 17 ist außerdem Teil einer Antriebseinrichtung, die den Behälter 10 im Gehäuse 2 in Richtung einer Längsachse 30 des Injektionsgeräts 1 verschiebt. Hierzu ist der Stößel 17 als Zahnstange ausgebildet und besitzt an mindestens zwei Seiten, die im Ausführungsbeispiel einander gegenüberliegend angeordnet sind, Zähne 18. In eine der Zahnreihen des Stößels 17 greift eine Klinke 16 ein, die an einem Halter 11 gelagert ist. In dem Halter 11 ist der Behälter 10 angeordnet. Die Klinke 16 bildet ein Koppelelement 39 zur Verbindung des Halters 11 mit dem Stößel 17. In die andere Zahnreihe, die in der schematischen Darstellung an der der Klinke 16 gegenüberliegenden Seite angeordnet ist, greift eine Klinke 20 ein, die an einem Antriebselement 19 gelagert ist. Die Zähne 18 des Stößels 20 sind sägezahnförmig ausgebildet und verlaufen an ihrer proximalen Flanke 68 flach und an ihrer distalen Flanke 69 steil, insbesondere etwa senkrecht zur Längsachse 30. Aufgrund der ungleichen Gestaltung der proximalen und distalen Flanken 68, 69 der Zähne 18 können der Halter 11 und das Antriebselement 19 nur in distaler Richtung gegenüber dem Stößel 17 verschoben werden. In Gegenrichtung ist die Bewegung aufgrund der Gestaltung der Zähne 18 gesperrt. Das Antriebselement 19 ist über eine Spannfeder 21 gegenüber dem Gehäuse 2 gefedert gelagert. Die Spannfeder 21 stützt sich an einer Schulter 33 des Antriebselements 19 ab. Am Antriebselement 19 ist ein Rastelement 60 angeordnet, das in eine Ausnehmung 29 des Gehäuses 2 einrasten kann. Das Antriebselement 19 bildet zusammen mit der Spannfeder 21, den Klinken 16 und 20 und dem Stößel 17 eine Antriebseinrichtung, die den Behälter 10 relativ zum Gehäuse 2 und den Stößel 17 relativ zum Behälter 10 bewegt.

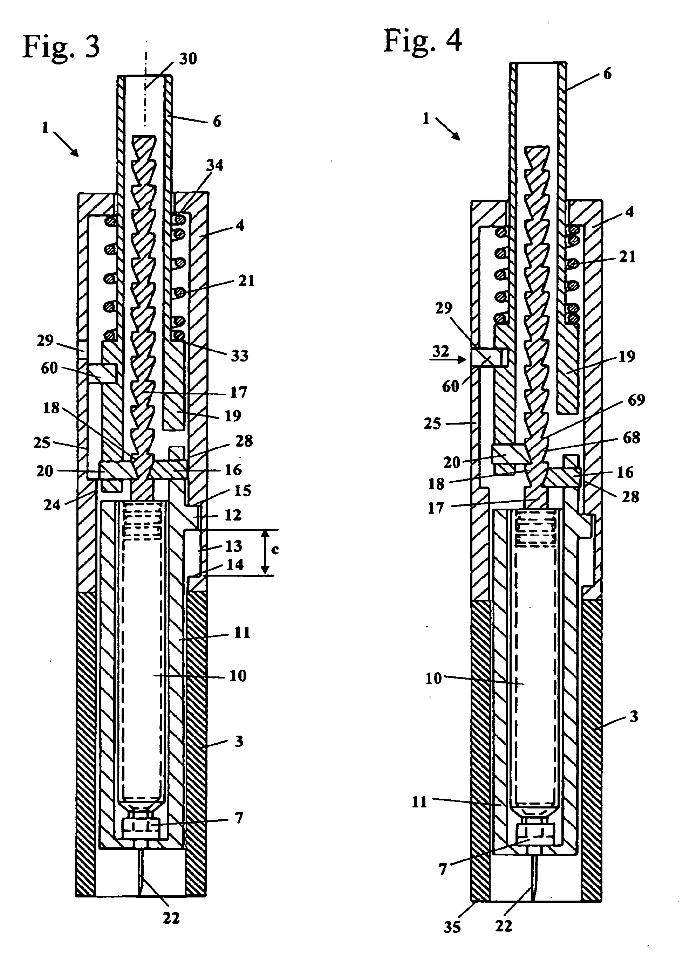

Um das Injektionsgerät 1 zu spannen, zieht der Bediener das Spannelement 6 in Richtung des Pfeils 31 in distale Richtung, also in Fig. 2 nach oben. Der gesamte Spannweg b ist der Weg, den das Rastelement 60 zurücklegt, bis es in die Ausnehmung 29 einrastet. Wird das Spannelement 6 aus der in Fig. 2 gezeigten Ruhestellung zurückgezogen, wird zunächst die gesamte Einheit aus Antriebselement 19, Stößel 17, Halter 11 und Behälter 10 im Gehäuse 2 verschoben. Dabei wird die Spannfeder 21 gespannt. Beim Zurückziehen des Spannelements 6 in Richtung des Pfeils 31 ist die Klinke 20 benachbart zu einer Sperrkontur 24 angeordnet, die verhindert, dass die Klinke 20 sich vom Stößel 17 weg nach außen bewegen und die Verrastung zwischen der Klinke 20 und dem Stößel 17 lösen kann. Über die Klinke 20 sind das Antriebselement 19 und der Stößel 17 in dieser Position fest miteinander verbunden. Eine Bewegung der Klinke 16 gegenüber dem Stößel 17 in proximaler Richtung ist aufgrund der Geometrie der Zähne 18 gesperrt. Beim Zurückziehen des Spannelements 6 aus der in Fig. 2 in die in Fig. 3 gezeigte Position kommt die Klinke 16 in dem Bereich einer Sperrkontur 28 (Fig. 3), die eine seitliche Bewegung der Klinke 20 und damit ein Lösen der Verrastung zwischen der Klinke 16 und dem Stößel 17 blockiert. Aufgrund der Sperrkonturen 24 und 28 sind Antriebselement 19, Stößel 17 und Halter 11 fest miteinander verbunden.

Wie die Figuren 2 und 3 zeigen, besitzt der Halter 11 einen nach außen ragenden Vorsprung 12, der in einer Ausnehmung 13 des Gehäuses 2 angeordnet ist. Bei der in Fig. 2 gezeigten Stellung ist der Vorsprung 12 an einem ersten, proximalen Anschlag 14 angeordnet. Beim Zurückziehen des Spannelements 6 bewegt sich der Vorsprung 12 zu einem zweiten, distalen Anschlag 15. Dabei legen der Halter 11 mit dem Behälter 10, der Stößel 17 und das Antriebselement 19 einen Stellweg c zurück, der dem Weg entspricht, um den die Kanüle 22 ins Gehäuse 2 gezogen wird. Fig. 3 zeigt das Injektionsgerät 1 nach dem Zurückziehen der Kanüle 22 ins Gehäuse 2. Beim weiteren Ziehen des Spannelements 6 aus der in Fig. 3 gezeigten Position in Richtung des Pfeils 31 in Fig. 2 wird eine weitere Bewegung des Halters 11 durch den Vorsprung 12, der am Anschlag 15 anliegt, blockiert. Über die Klinke 16 ist der Stößel 17 fest mit dem Halter 11 verbunden, so dass auch der Stößel 17 nicht weiter in distaler Richtung bewegt werden kann. Die Klinke 20 ist bei der in Fig. 3 gezeigten Stellung im Bereich einer Ausnehmung 25 am Gehäuse 2 angeordnet und kann deshalb radial nach außen gleiten und sich bei weiterem Herausziehen des Spannelements 6 gegenüber dem Stößel 17 um einen Zahn 18 in distaler Richtung bewegen. Dabei wird die Spannfeder 21 weiter gespannt, bis das Antriebselement 19 in die in Fig. 4 gezeigte Position gelangt. Sobald sich das Antriebselement 19 um einen Zahn 18 gegenüber dem Stößel 17 bewegt hat, rastet das Rastelement 60 in der Ausnehmung 29 des Gehäuses 2 ein, so dass keine weitere distale Bewegung des Spannelements 6 mehr möglich ist.

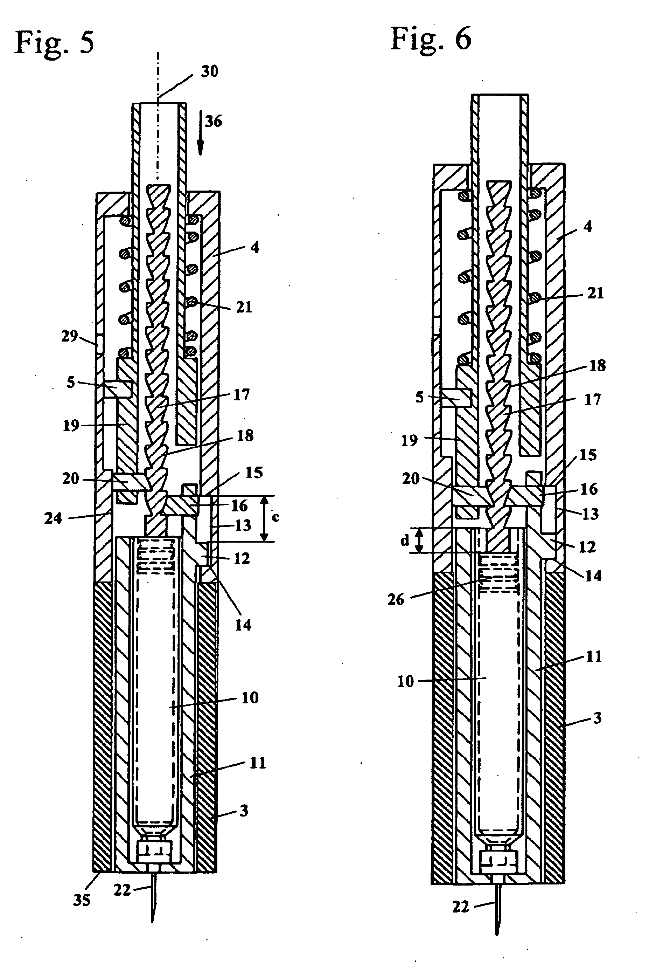

Fig. 4 zeigt das Injektionsgerät 1 in vollständig gespannter Stellung. In dieser Stellung setzt der Bediener das Injektionsgerät 1 mit der Auflage 35 auf die Haut auf und betätigt das Rastelement 60 in Richtung des Pfeils 32. Dies erfolgt vorteilhaft über das Auslöseelement 5, das in den Figuren 2 bis 6 nicht gezeigt ist. Dadurch wird die Verrastung zwischen Rastelement 60 und Ausnehmung 29 gelöst. Aufgrund der in der Spannfeder 21 gespeicherten Energie wird das Antriebselement 19 in Richtung des in Fig. 5 gezeigten Pfeils 36 bewegt. Dabei nimmt das Antriebselement 19 den Stößel 17 über die Klinke 20 mit. Die Klinke 20 hat sich von der Ausnehmung 25 zur Sperrkontur 24 bewegt und kann in radialer Richtung nicht ausweichen. Die Klinke 16 ist im Bereich der Sperrkontur 28 angeordnet und kann ebenfalls nicht ausweichen, so dass auch der Halter 11 und der Behälter 10 sowie die Injektionsnadel 7 in proximaler Richtung bewegt werden. Eine Relativbewegung von Antriebselement 19 und Halter 11 in distaler Richtung gegenüber dem Stößel 17 ist auch aufgrund der Gestaltung der distalen Flanken 69 der Zähne 18 nicht möglich.

Bei der Bewegung des Halters 11 tritt die Kanüle 22 am proximalen Ende aus dem Gehäuse 2 aus und sticht in die Haut des Bedieners ein. Bei dieser Bewegung des Halters 11 relativ zum Gehäuse 2 bewegt sich der Vorsprung 12 in der Ausnehmung 13 vom distalen Anschlag 15 zum proximalen Anschlag 14, und zwar um den Stellweg c. Diese Position ist in Fig. 5 gezeigt. Aufgrund der in der Spannfeder 21 gespeicherten Kraft wird das Antriebselement 19 weiter in proximaler Richtung bewegt. Die Klinke 16 ist nach dem Einstichvorgang im Bereich der Ausnehmung 13 angeordnet und kann sich gegenüber dem Stößel 17 nach außen bewegen und am nächsten Zahn 18 einrasten. Der Halter 11 kann sich aufgrund der Anlage des Vorsprungs 12 am Anschlag 14 nicht weiter bewegen. Das Antriebselement 19 nimmt bei seiner proximalen Bewegung aus der in Fig. 5 gezeigten Position in die in Fig. 6 gezeigte Position den Stößel 17 mit, der sich relativ zum Behälter 10 bewegt und den Stopfen 26 im Behälter 10 verschiebt und dadurch Injektionsflüssigkeit durch die Kanüle 22 auspresst. Dabei legt der Stößel 17 einen Dosierweg d zurück, der dem Abstand der distalen Stirnseite von zwei aufeinander folgenden Zähnen 18 entspricht. Der Dosierweg d und der Stellweg c entsprechen zusammen dem in Fig. 2 gezeigten Spannweg b. Bei Injektionsgeräten, bei denen die Menge der zu injizierenden Flüssigkeit einstellbar ist, kann der Dosierweg d vom Bediener eingestellt werden.

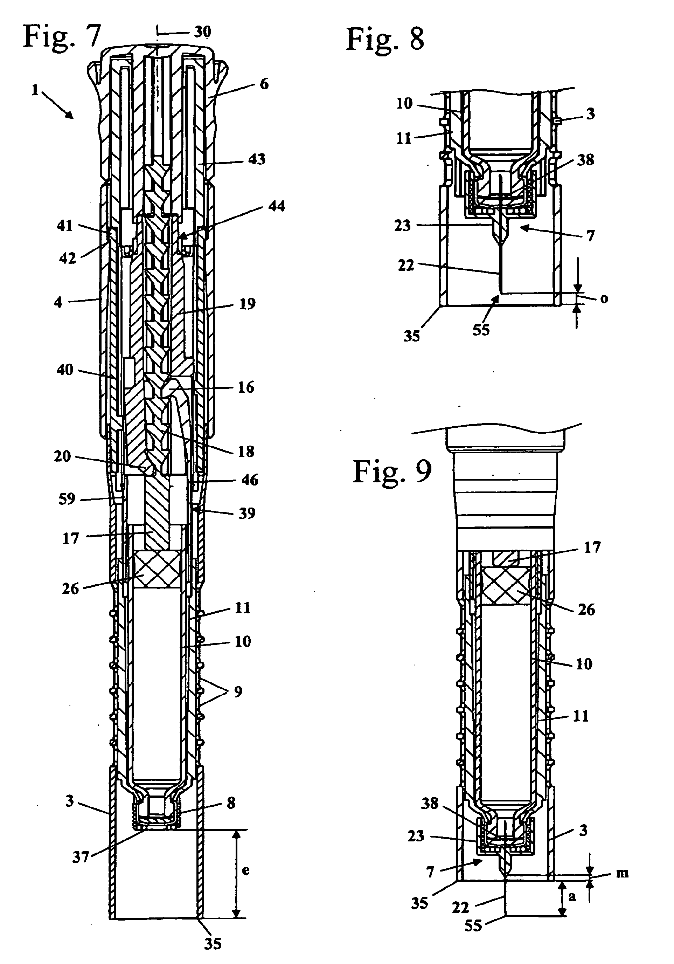

Fig. 7 zeigt die konstruktive Gestaltung des Injektionsgeräts 1. Fig. 7 zeigt das Injektionsgerät 1 in teilweise gespanntem Zustand, in dem der Behälter 10 in seiner distalen Endlage angeordnet ist, das Antriebselement 19 aber noch nicht gegenüber dem Stößel 17 in distaler Richtung verschoben wurde. Dies entspricht der in Fig. 3 gezeigten Position. Das Spannelement 6 ist an einer Führungshülse 43 geführt, die fest mit dem zweiten Gehäuseteil 4 verbunden ist. Mit dem zweiten Gehäuseteil 4 ist außerdem eine Hülse 40 fest verbunden. Die Hülse 40 und das zweite Gehäuseteil 4 könnten auch einteilig ausgebildet sein. Aufgrund der zweiteiligen Ausbildung wird die Herstellung vereinfacht. Die Hülse 40 liegt mit einem radial nach außen ragenden Rand 41 an einem Absatz 42 des Gehäuseteils 4 an. Das Spannelement 6 ist an einer Verbindung 44 fest mit dem Antriebselement 19 verbunden. Auch hier kann eine einteilige Ausbildung der beiden Elemente vorgesehen sein. Im Inneren des Antriebselements 19 ist der Stößel 17 geführt. Am Antriebselement 19 ist die Klinke 20 ausgebildet. Das Antriebselement 19 ist aus Kunststoff und die Klinke 20 ist am Antriebselement 19 angeformt und als in Richtung der Längsachse 30 auskragender Arm ausgebildet. Aufgrund der Eigenelastizität des Materials ist die Klinke 20 federnd ausgebildet.

An der der Klinke 20 gegenüberliegenden Seite ist die Klinke 16 angeordnet, die an einem zylindrischen Abschnitt 59 des Koppelelements 39 angeformt ist. Auch das Koppelelement 39 besteht aus Kunststoff, und die Klinke 16 ist an einem aufgrund der Eigenelastizität des Materials federnden Arm ausgebildet.

Die Hülse 40 besitzt an ihrem proximalen Ende Rastfinger 46, an denen das Koppelelement 39 eingerastet ist. Das Koppelelement 39 ist dabei in proximaler Richtung gegenüber der Hülse 40 beweglich. Das Koppelelement 39 ist fest mit dem Halter 11 verbunden. Der Stößel 17 liegt am Stopfen 26 an. Wie Fig. 7 zeigt, besitzt der Halter 11 an seinem proximalen Ende eine Aufnahme 8 für die Injektionsnadel 7, die als Außengewinde ausgebildet ist, auf das die Injektionsnadel 7 mit ihrem Kanülenhalter 23 aufgeschraubt werden kann. Die proximale Stirnseite 37 der Aufnahme 8 besitzt in der in Fig. 7 gezeigten, zurückgezogenen Stellung des Halters 11 einen Abstand e zur Auflage 35.

Fig. 8 zeigt die Anordnung aus Fig. 7 mit an der Aufnahme 8 angeordneter Injektionsnadel 7. Die Injektionsnadel 7 besitzt an ihrem proximalen Ende eine Spitze 55, die innerhalb des ersten Gehäuseteils 3 angeordnet ist. Die Spitze 55 ragt demnach nicht über die Auflage 35 hinaus. Die Spitze 55 besitzt zur Auflage 35 einen Abstand o. Der Abstand o kann dabei sehr klein sein. Wie Fig. 8 auch zeigt, ragt die Kanüle 22 durch eine Membran 38 des Behälters 10 in den Innenraum des Behälters 10.

Fig. 9 zeigt das Injektionsgerät 1 im Ruhezustand. In diesem Zustand ist der Behälter 10 in seiner proximalen Endlage angeordnet, und die Kanüle 22 der Injektionsnadel 7 ragt über die Auflage 35 hinaus. In diesem Zustand befindet sich das Injektionsgerät 1 nach einer Injektion, bevor die Injektionsnadel 7 entfernt wurde, und vor dem Spannen des Injektionsgeräts 1, nachdem eine neue Injektionsnadel 7 aufgesetzt wurde. In diesem Ruhezustand ragt die Kanüle 22 über die Auflage 35 hinaus. Die Spitze 55 der Kanüle 22 besitzt zur Auflage 35 den Abstand a. Der Kanülenhalter 23 ist vollständig innerhalb des Gehäuseteils 3 angeordnet und besitzt zur Auflage 35 einen Abstand m. Dadurch wird verhindert, dass der Kanülenhalter 23 mit dem Bediener in Kontakt kommen kann.

Die Figuren 9 bis 15 zeigen den Ablauf bei der Herstellung des Injektionsgeräts 1. Die Montage der Antriebseinrichtung ist dabei nicht im Einzelnen gezeigt. Diese ist bereits montiert und im zweiten Gehäuseteil 4 angeordnet. Dabei ragt die Hülse 40 über die proximale Stirnseite 34 des zweiten Gehäuseteils 4 hinaus. Am proximalen Ende der Hülse 40 sind die Rastfinger 46 angeordnet, an denen der zylindrische Abschnitt 59 des Koppelelements 39 eingerastet ist. Der zylindrische Abschnitt 59 besitzt einen Anschlag 45. Zwischen dem Anschlag 45 und dem proximalen Ende 52 des Koppelelements 39 besitzt das Koppelelement 39 einen Rand 47, der parallel zur Längsachse 30 verlaufende Längsstege 48 besitzt. Die Längsstege 48 sind als Erhöhungen ausgebildet, so dass zwischen den Längsstegen 48 Taschen gebildet sind, die zur Aufnahme von Klebstoff dienen können.

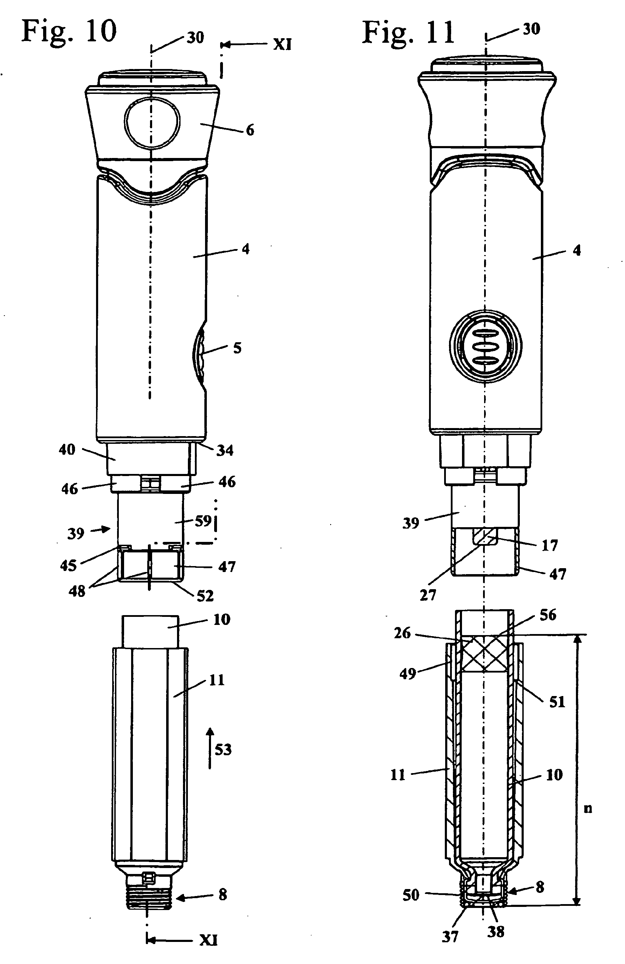

Bei der Montage wird zunächst der Behälter 10 im Halter 11 angeordnet. Anschließend wird der Halter 11 in Richtung des Pfeils 53 auf den Rand 47 aufgeschoben. Vor dem Aufschieben des Halters 11 wird am Rand 47 Klebstoff angeordnet, wenn der Halter 11 am Koppelelement 39 verklebt werden soll. Der Behälter 10 ragt ins Innere des Rands 47, und der Halter 11 übergreift den Rand 47. Wie Fig. 11 zeigt, ist zwischen dem Halter 11 und dem Behälter 10 ein Ringraum 49 gebildet, der in Längsrichtung 30 von einer Schulter 51 am Halter 11 begrenzt wird. Die Schulter 51 und der Anschlag 45 begrenzen die maximale Einschubtiefe. Der Halter 11 wird so weit auf den Rand 47 aufgeschoben, bis die Stirnseite 56 des Stopfens 26 an der Stirnseite 27 des Stößels 17 anliegt. Die Position des Stopfens 26 im Behälter 10 ist vergleichsweise großen Toleranzen unterworfen. Die Position des Stopfens 26 kann beispielsweise von Behälter zu Behälter um etwa einen Millimeter schwanken. Die Stirnseite 56 besitzt zur Stirnseite 37 der Aufnahme 8 einen Abstand n, der entsprechend der Position des Stopfens 26 variiert. Wie Fig. 11 zeigt, besitzt der Halter 11 an seinem proximalen Ende ein Gewinde 50, das die Aufnahme 8 bildet. Das Gewinde 50 ist so ausgebildet, dass der Kanülenhalter 23 (Fig. 9) bis an die Stirnseite 37 des Halters 11 aufgeschraubt werden kann. Dadurch ist die Position der Spitze 55 der Injektionsnadel 7 gegenüber dem Halter 11 definiert.

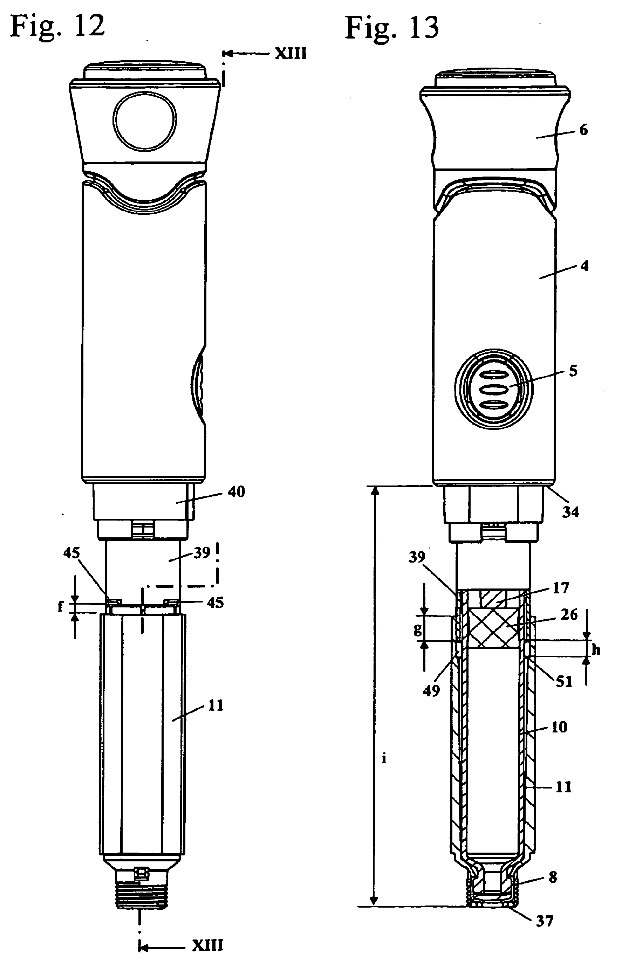

Nachdem der Halter 11 auf das Koppelelement 39 aufgeschoben wurde, werden der Halter 11 und das Koppelelement 39 fest und unlösbar miteinander verbunden. Dies kann beispielsweise durch Kleben erfolgen. Der Halter 11 ist vorteilhaft durchsichtig, und die Verklebung erfolgt mit einem Klebstoff, der unter Strahlung, insbesondere unter UV-Strahlung aushärtet. Es kann auch eine unlösbare Verbindung durch Verpressen, insbesondere durch Heißpressen, oder durch Verschweißen, insbesondere durch Laserschweißen oder durch Ultraschallschweißen, vorgesehen sein. Die Figuren 12 und 13 zeigen die Position des Halters 11 nach der Positionierung und Fixierung am Koppelelement 39. Der Anschlag 45 besitzt zum Halter 11 einen Abstand f. Das Koppelelement 39 ragt um eine Einschubtiefe g in den Ringraum 49. Das Koppelelement 39 besitzt dabei zur Schulter 51 einen Abstand h. Die Abmessungen des Ringraums 49 und die Position des Anschlags 45 sind so gewählt, dass bei allen auftretenden Toleranzen sowohl zwischen Halter 11 und Anschlag 45 der Abstand f als auch zwischen Koppelelement 39 und der Schulter 51 der Abstand h gebildet ist. Die proximale Stirnseite 37 des Halters 11 besitzt zur proximalen Stirnseite 34 des Gehäuseteils 4 einen Abstand i, der von der Position des Stopfens 26 abhängig ist.

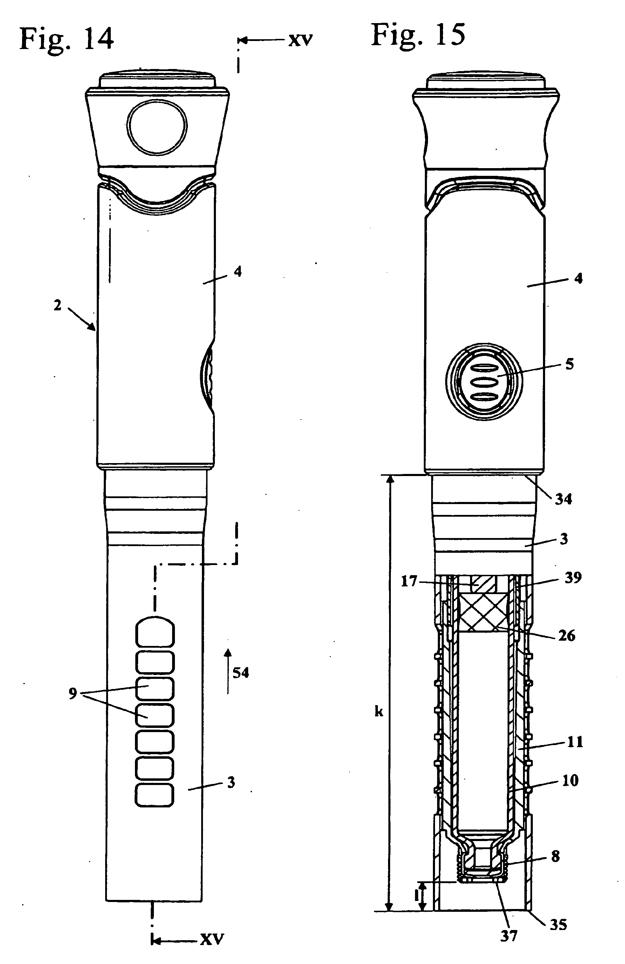

Nach der Fixierung des Halters 11 wird das zweite Gehäuseteil 4 am ersten Gehäuseteil 3 positioniert und an diesem festgelegt. Dies ist in den Figuren 14 und 15 gezeigt. Das erste Gehäuseteil 3 wird in Richtung des Pfeils 54 von der proximalen Seite in das zweite Gehäuseteil 4 eingeschoben. Das zweite Gehäuseteil 4 übergreift dabei das erste Gehäuseteil 3. Die Positionierung des ersten Gehäuseteils 3 am zweiten Gehäuseteil 4 erfolgt derart, dass sich ein definierter Abstand 1 zwischen der Stirnseite 37 des Halters 11 und der Auflage 35 ergibt. Dieser Abstand in der in Fig. 15 gezeigten proximalen Position des Halters 11 und des Behälters 10 ist so gewählt, dass sich für eine an der Aufnahme 8 angeordnete Injektionsnadel 7 eine gewünschte Einstichtiefe ergibt. Der Kanülenhalter 23 ist in dieser Position vorteilhaft innerhalb des ersten Gehäuseteils 3 angeordnet. Die Positionierung des ersten Gehäuseteils 3 erfolgt durch Veränderung des Abstands k zwischen der Auflage 35 und der proximalen Stirnseite 34 des zweiten Gehäuseteils 4. Sobald der gewünschte Abstand 1 eingestellt ist, wird das erste Gehäuseteil 3 am zweiten Gehäuseteil 4 unlösbar befestigt. Die Befestigung erfolgt vorteilhaft durch Verkleben, Verpressen, insbesondere Heißpressen, oder Schweißen, insbesondere Laserschweißen oder Ultraschallschweißen.

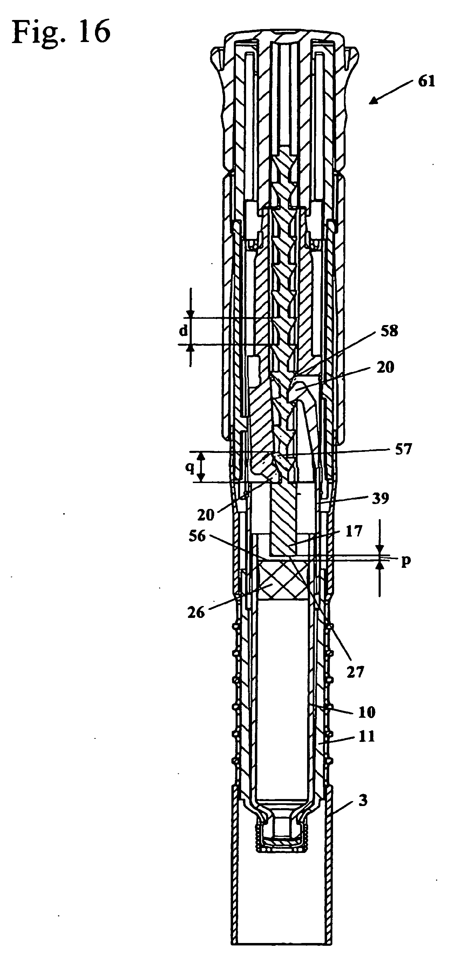

Bei dem in Fig. 16 gezeigten Ausführungsbeispiel eines Injektionsgeräts 61 liegt die Stirnseite 27 des Stößels 17 nicht an der Stirnseite 56 des Stopfens 26 an, sondern besitzt zu dieser einen definierten Abstand p. Das Aufschieben des Halters 11 auf das Koppelelement 39 erfolgt bei diesem Ausführungsbeispiel nicht, bis der Stößel 17 am Stopfen 26 anliegt, sondern bis der definierte Abstand p erreicht ist. Um sicherzustellen, dass bei der ersten Injektion der Stopfen 26 um den gewünschten Dosierweg d bewegt wird, besitzt der erste Zahn 57, über den die Klinke 20 gleitet, eine Höhe q, die größer als der Dosierweg d ist, und zwar um den Abstand p größer als dieser. Dadurch wird der Stößel 17 beim ersten Injektionsvorgang um den Abstand p bis zum Stopfen 26 und zusätzlich um den Dosierweg d verstellt. Entsprechend ist der erste Zahn 58, über den die Klinke 20 gleitet, vergrößert ausgeführt, so dass der Stößel 17 nach dem ersten Injektionsvorgang am Stopfen 26 anliegt.

## Patentansprüche

1. Verfahren zur Herstellung eines Injektionsgeräts, wobei das Injektionsgerät (1, 61) ein Gehäuse (2) besitzt, das ein erstes Gehäuseteil (3) und ein zweites Gehäuseteil (4) umfasst, wobei das Injektionsgerät (1,61) eine Aufnahme (8) für eine Injektionsnadel (7) besitzt, wobei die Aufnahme (8) mit einer Antriebseinrichtung verbunden ist, die zur Bewegung der Aufnahme (8) relativ zu dem zweiten Gehäuseteil (4) zwischen einer distalen und einer proximalen Endlage dient, und wobei das erste Gehäuseteil (3) eine Auflage (35) zum Aufsetzen auf die Haut vor dem Auslösen einer Injektion besitzt,
**dadurch gekennzeichnet, dass** das erste Gehäuseteil (3) an dem zweiten Gehäuseteil (4) so positioniert wird, dass die Aufnahme (8) in ihrer proximalen Endlage eine vorgegebene Lage relativ zu der Auflage (35) besitzt, und dass das erste Gehäuseteil (3) in dieser Position gegenüber dem zweiten Gehäuseteil (4) unlösbar fixiert wird.

2. Verfahren nach Anspruch 1,
**dadurch gekennzeichnet, dass** das Injektionsgerät (1,61) einen Behälter (10) mit Injektionsflüssigkeit besitzt, in dem ein beweglicher Stopfen (26) angeordnet ist, dass die Antriebseinrichtung einen Stößel (17) besitzt, der auf den Stopfen (26) wirkt, dass der Behälter (10) relativ zu dem Stößel (17) so positioniert wird, dass sich eine definierte Lage des Stopfens (26) relativ zu dem Stößel (17) ergibt, und dass der Behälter (10) in dieser Position fixiert wird.

3. Verfahren nach Anspruch 2,
**dadurch gekennzeichnet, dass** die Positionierung des Behälters (10) relativ zu dem Stößel (17) vor der Positionierung des ersten Gehäuseteils (3) relativ zu dem zweiten Gehäuseteil (4) erfolgt.

4. Verfahren nach Anspruch 2 oder 3,
**dadurch gekennzeichnet, dass** das Injektionsgerät (1, 61) ein Koppelelement (39) besitzt, das mit dem Stößel (17) zusammenwirkt, wobei der Behälter (10) nach der Positionierung relativ zu dem Stößel (17) gegenüber dem Koppelelement (39) fixiert wird.

5. Verfahren nach Anspruch 4,
**dadurch gekennzeichnet, dass** der Behälter (10) in einem Halter (11) angeordnet wird, der die Aufnahme (8) trägt, und dass der Halter (11) mit dem darin angeordneten Behälter (10) an dem Koppelelement (39) fixiert wird.

6. Verfahren nach einem der Ansprüche 1 bis 5,
**dadurch gekennzeichnet, dass** das erste Gehäuseteil (3) mit dem zweiten Gehäuseteil (4) verklebt, verpresst oder verschweißt wird.

7. Injektionsgerät mit einem Gehäuse (2), das ein erstes Gehäuseteil (3) und ein zweites Gehäuseteil (4) umfasst, wobei das Injektionsgerät (1,61) eine Aufnahme (8) für eine Injektionsnadel (7) besitzt, wobei die Aufnahme (8) mit einer Antriebseinrichtung verbunden ist, die zur Bewegung der Aufnahme (8) relativ zu dem zweiten Gehäuseteil (4) zwischen einer distalen und einer proximalen Endlage dient, und wobei das erste Gehäuseteil (3) eine Auflage (35) zum Aufsetzen auf die Haut vor dem Auslösen einer Injektion besitzt,
**dadurch gekennzeichnet, dass** das erste Gehäuseteil (3) gegenüber dem zweiten Gehäuseteil (4) unlösbar fixiert ist.

8. Injektionsgerät nach Anspruch 7,
**dadurch gekennzeichnet, dass** das erste Gehäuseteil (3) und das zweite Gehäuseteil (4) miteinander verklebt, verpresst oder verschweißt sind.

9. Injektionsgerät nach Anspruch 7 oder 8,
**dadurch gekennzeichnet, dass** das Injektionsgerät (1, 61) einen Behälter (10) mit Injektionsflüssigkeit besitzt, in dem ein beweglicher Stopfen (26) angeordnet ist, und dass die Antriebseinrichtung einen Stößel (17) besitzt, der auf den Stopfen (26) wirkt.

10. Injektionsgerät nach Anspruch 9,
**dadurch gekennzeichnet, dass** der Stößel (17) vor der ersten Injektion an dem Stopfen (26) anliegt.

11. Inj ektionsgerät nach Anspruch 10,
**dadurch gekennzeichnet, dass** der Stößel (17) vor der ersten Injektion zu dem Stopfen (26) einen Abstand (p) besitzt, wobei die Antriebseinrichtung so ausgebildet ist, dass der Stößel (17) bei der ersten Injektion einen Weg zurücklegt, der um den Abstand (p) zwischen Stößel (17) und Stopfen (26) größer als der zum Herausdrücken der eingestellten Menge an Injektionsflüssigkeit benötigte Weg ist.

12. Injektionsgerät nach einem der Ansprüche 9 bis 11,
**dadurch gekennzeichnet, dass** der Behälter (10) mit einem Koppelelement (39) verbunden ist, das mit dem Stößel (17) zusammenwirkt und den Stößel (17) je nach Betriebszustand relativ zu dem Behälter (10) fixiert oder gegenüber dem Behälter (10) freigibt.

13. Injektionsgerät nach Anspruch 12,
**dadurch gekennzeichnet, dass** der Behälter (10) in einem Halter (11) angeordnet ist, der die Aufnahme (8) für die Injektionsnadel (7) besitzt.

14. Injektionsgerät nach Anspruch 13,
**dadurch gekennzeichnet, dass** der Halter (11) aus einem durchsichtigen Material besteht und mit dem Koppelelement (39) über eine durch Strahlung aushärtende Klebverbindung verbunden ist.

15. Injektionsgerät nach einem der Ansprüche 9 bis 14,
**dadurch gekennzeichnet, dass** das Injektionsgerät (1,61) eine Spannfeder (21) besitzt, die beim Injektionsvorgang sowohl eine Bewegung des Behälters (10) relativ zu dem Gehäuse (2) als auch eine Bewegung des Stößels (17) relativ zu dem Behälter (10) bewirkt.

16. Injektionsgerät nach einem der Ansprüche 9 bis 15,
**dadurch gekennzeichnet, dass** die Antriebseinrichtung über eine Koppeleinrichtung mit dem Behälter (10) und dem Stößel (17) verbunden ist, wobei die Koppeleinrichtung in Abhängigkeit der Position von Antriebseinrichtung, Behälter (10) und Stößel (17) relativ zu dem Gehäuse (2) gesteuert ist.

## Claims

1. Method for producing an injection device, wherein the injection device (1, 61) has a housing (2) which comprises a first housing part (3) and a second housing part (4), wherein the injection device (1, 61) has a receiving part (8) for an injection needle (7), wherein the receiving part (8) is connected to a drive means which is used to move the receiving part (8) relative to the second housing part (4) between a distal and a proximal end position, and wherein the first housing part (3) has a support (35) for placing on the skin before initiating an injection,
**characterised in that** the first housing part (3) is positioned on the second housing part (4) so that the receiving part (8) has in its proximal end position a predefined position relative to the support (35), and the first housing part (3) in this position is non-detachably fixed with respect to the second housing part (4).

2. Method according to claim 1,
**characterised in that** the injection device (1, 61) has a container (10) with injection liquid, in which a movable stopper (26) is arranged, the drive means has a plunger (17) which acts on the stopper (26), the container (10) is positioned relative to the plunger (17) so that a defined position of the stopper (26) relative to the plunger (17) is produced and the container (10) is fixed in this position.

3. Method according to claim 2,
**characterised in that** the positioning of the container (10) relative to the plunger (17) is realised before the positioning of the first housing part (3) relative to the second housing part (4).

4. Method according to claim 2 or 3,
**characterised in that** the injection device (1, 61) has a coupling element (39) which cooperates with the plunger (17), wherein the container (10) after positioning relative to the plunger (17) is fixed with respect to the coupling element (39).

5. Method according to claim 4,
**characterised in that** the container (10) is arranged in a holder (11) which carries the receiving part (8), and the holder (11) is fixed with the container (10) arranged therein to the coupling element (39).

6. Method according to one of claims 1 to 5,
**characterised in that** the first housing part (3) is bonded, pressed or welded to the second housing part (4).

7. Injection device with a housing (2) which comprises a first housing part (3) and a second housing part (4), wherein the injection device (1, 61) has a receiving part (8) for an injection needle (7), wherein the receiving part (8) is connected to a drive means which is used to move the receiving part (8) relative to the second housing part (4) between a distal and a proximal end position, and wherein the first housing part (3) has a support (35) for placing on the skin before initiating an injection, **characterised in that** the first housing part (3) is fixed non-detachably with respect to the second housing part (4).

8. Injection device according to claim 7,
**characterised in that** the first housing part (3) and the second housing part (4) are bonded, pressed or welded to each other.

9. Injection device according to claim 7 or 8,
**characterised in that** the injection device (1,61) has a container (10) with injection liquid, in which a movable stopper (26) is arranged, and the drive means has a plunger (17) which acts on the stopper (26).

10. Injection device according to claim 9,
**characterised in that** the plunger (17) lies against the stopper (26) before the first injection.

11. Injection device according to claim 10,
**characterised in that** before the first injection the plunger (17) is at a distance (p) from the stopper (26), wherein the drive means is formed so that the plunger (17) at the time of the first injection is set back by an amount which is greater, by the distance (p) between the plunger (17) and the stopper (16), than the amount required to push out the set amount of injection liquid.

12. Injection device according to one of claims 9 to 11,
**characterised in that** the container (10) is connected to a coupling element (39) which cooperates with the plunger (17) and, subject to the operating state, fixes the plunger (17) relative to the container (10) or releases it with respect to the container (10).

13. Injection device according to claim 12,
**characterised in that** the container (10) is arranged in a holder (11) which has the receiving part (8) for the injection needle (7).

14. Injection device according to claim 13,
**characterised in that** the holder (11) is made of a transparent material and is connected to the coupling element (39) via a bonding connection that hardens through radiation.

15. Injection device according to one of claims 9 to 14,
**characterised in that** the injection device (1, 61) has a clamping spring (21) which causes, during the injection process, both a movement of the container (10) relative to the housing (2) and also a movement of the plunger (17) relative to the container (10).

16. Injection device according to one of claims 9 to 15,
**characterised in that** the drive means is connected via a coupling means to the container (10) and the plunger (17), wherein the coupling means is controlled in dependence upon the position of the drive means, container (10) and plunger (17) relative to the housing (2).

## Revendications

1. Procédé pour fabriquer un dispositif d'injection, étant précisé que le dispositif d'injection (1,61) comporte un boîtier (2) qui comprend un premier élément de boîtier (3) et un second élément de boîtier (4), étant précisé que le dispositif d'injection (1, 61) comporte un logement (8) pour une aiguille d'injection (7), que le logement (8) est relié à un dispositif d'entraînement qui sert à déplacer ledit logement (8) par rapport au second élément de boîtier (4) entre une position de fin de course distale et une position de fin de course proximale, et que le premier élément de boîtier (3) comporte une partie d'appui (35) à poser sur la peau avant le déclenchement d'une injection,
**caractérisé en ce que** le premier élément de boîtier (3) est positionné sur le second élément de boîtier (4) de telle sorte que le logement (8), dans sa position de fin de course proximale, présente par rapport à la partie d'appui (35) une position prédéfinie, et **en ce que** le premier élément de boîtier (3) est fixé de manière non amovible dans cette position par rapport au second élément de boîtier (4).

2. Procédé selon la revendication 1,
**caractérisé en ce que** le dispositif d'injection (1, 61) comporte un récipient (10) avec un liquide d'injection, dans lequel est disposé un bouchon mobile (26), **en ce que** le dispositif d'entraînement comporte un piston (17) qui agit sur le bouchon (26), **en ce que** le récipient (10) est positionné par rapport au piston (17) de telle sorte qu'on obtient une position définie du bouchon (26) par rapport au piston (17), et **en ce que** le récipient (10) est fixé dans cette position.

3. Procédé selon la revendication 2,
**caractérisé en ce que** le positionnement du récipient (10) par rapport au piston (17) se fait avant le positionnement du premier élément de boîtier (3) par rapport au second élément de boîtier (4).

4. Procédé selon la revendication 2 ou 3,
**caractérisé en ce que** le dispositif d'injection (1, 61) comporte un élément d'accouplement (39) qui coopère avec le piston (17), étant précisé que le récipient (10), une fois positionné par rapport au piston (17), est fixé par rapport à l'élément d'accouplement (39).

5. Procédé selon la revendication 4,
**caractérisé en ce que** le récipient (10) est disposé dans un support (11) qui porte le logement (8), et **en ce que** le support (11), avec le récipient (10) qu'il renferme, est fixé à l'élément d'accouplement (39).

6. Procédé selon l'une des revendications 1 à 5,
**caractérisé en ce que** le premier élément de boîtier (3) est relié au second élément de boîtier (4) par collage, pression ou soudure.

7. Dispositif d'injection avec un boîtier (2) qui comprend un premier élément de boîtier (3) et un second élément de boîtier (4), étant précisé que le dispositif d'injection (1, 61) comporte un logement (8) pour une aiguille d'injection (7), que le logement (8) est relié à un dispositif d'entraînement qui sert à déplacer ledit logement (8) par rapport au second élément de boîtier (4) entre une position de fin de course distale et une position de fin de course proximale, et que le premier élément de boîtier (3) comporte une partie d'appui (35) à poser sur la peau avant le déclenchement d'une injection,
**caractérisé en ce que** le premier élément de boîtier (3) est fixé de manière non amovible par rapport au second élément de boîtier (4).

8. Dispositif d'injection selon la revendication 7,
**caractérisé en ce que** le premier élément de boîtier (3) et le second élément de boîtier (4) sont reliés par collage, pression ou soudure.

9. Dispositif d'injection selon la revendication 7 ou 8,
**caractérisé en ce que** le dispositif d'injection (1,61) comporte un récipient (10) avec un liquide d'injection, dans lequel est disposé un bouchon mobile (26), et **en ce que** le dispositif d'entraînement comporte un piston (17) qui agit sur le bouchon (26).

10. Dispositif d'injection selon la revendication 9,
**caractérisé en ce que** le piston (17), avant la première injection, est appliqué contre le bouchon (26).

11. Dispositif d'injection selon la revendication 10,
**caractérisé en ce que** le piston (17), avant la première injection, présente un écartement (p) par rapport au bouchon (26), étant précisé que le dispositif d'entraînement est conçu pour que le piston (17), lors de la première injection, parcoure une distance qui est plus grande de la valeur (p) d'écartement entre le piston (17) et le bouchon (26), que la distance nécessaire pour expulser la quantité réglée de liquide d'injection.

12. Dispositif d'injection selon l'une des revendications 9 à 11,
**caractérisé en ce que** le récipient (10) est relié à l'élément d'accouplement (39) qui coopère avec le piston (17) et qui, suivant l'état de fonctionnement, fixe ou libère le piston (17) ledit récipient (10).

13. Dispositif d'injection selon la revendication 12,
**caractérisé en ce que** le récipient (10) est disposé dans un support (11) qui comporte un logement (8) pour l'aiguille d'injection (7).

14. Dispositif d'injection selon la revendication 13,
**caractérisé en ce que** le support (11) se compose d'un matériau transparent et est relié à l'élément d'accouplement (39) grâce à une liaison par collage qui durcit par rayonnement.

15. Dispositif d'injection selon l'une des revendications 9 à 14,
**caractérisé en ce que** le dispositif d'injection (1,61) comporte un ressort de tension (21) qui, lors de l'injection, provoque aussi bien un déplacement du récipient (10) par rapport au boîtier (2) qu'un déplacement du piston (17) par rapport au récipient (10).

16. Dispositif d'injection selon l'une des revendications 9 à 15,
**caractérisé en ce que** le dispositif d'entraînement est lié au récipient (10) et au piston (17) par l'intermédiaire d'un dispositif d'accouplement, étant précisé que le dispositif d'accouplement est commandé en fonction de la position du dispositif d'entraînement, du récipient (10) et du piston (17) par rapport au boîtier (2).
